## Europäisches Patentamt
## European Patent Office
## Office européen des brevets

(11) Veröffentlichungsnummer: **0 182 888 B1**

(12) **EUROPÄISCHE PATENTSCHRIFT**

(45) Veröffentlichungstag der Patentschrift: **04.12.91**

(21) Anmeldenummer: **85902983.7**

(22) Anmeldetag: **05.06.85**

(86) Internationale Anmeldenummer: **PCT/EP85/00273**

(87) Internationale Veröffentlichungsnummer: **WO 85/05690 (19.12.85 85/27)**

Die Akte enthält technische Angaben, die nach dem Eingang der Anmeldung eingereicht wurden und die nicht in dieser Patentschrift enthalten sind.

(51) Int. Cl.⁵: **G01N 33/573**, G01N 33/577, C12P 21/00, C12N 15/00, C12N 5/00, //(C12P21/00, C12R1:91)

(54) **VERFAHREN UND REAGENZ ZUR DIFFERENZIERTEN BESTIMMUNG VON ISOENZYMEN DER ALKALISCHEN PHOSPHATASE SOWIE HIERZU GEEIGNETER MONOKLONALER ANTIKÖRPER.**

(30) Priorität: **05.06.84 DE 3420926**

(43) Veröffentlichungstag der Anmeldung:
**04.06.86 Patentblatt 86/23**

(45) Bekanntmachung des Hinweises auf die Patenterteilung:
**04.12.91 Patentblatt 91/49**

(84) Benannte Vertragsstaaten:
**AT BE CH DE FR GB IT LI LU NL SE**

(56) Entgegenhaltungen:
**EP-A- 32 204**
**EP-A- 93 436**

**WO 84/02720**

(73) Patentinhaber: **BOEHRINGER MANNHEIM GMBH**
**Sandhofer Strasse 116**
**W-6800 Mannheim 31(DE)**

(72) Erfinder: **KATZMANN, Jerry, Allen**
**1517 SW 6th Street**
**Rochester, MN 55902(US)**
Erfinder: **LAWSON, George, Mathews**
**109 W Kelling Road**
**Greensboro, NC 27410(US)**
Erfinder: **O'BRIEN, John, Frank**
**RR 1, Box 25**
**Byron, MN 55920(US)**

(74) Vertreter: **Daum, Martin, Dr. et al**
**Boehringer Mannheim GmbH. Sandhofer-strasse 116**
**W-6800 Mannheim 31(DE)**

CLIN.CHEM., vol. 31, no. 3, March 1985; G.LAWSON et al., pp. 381-385 : "Isolation and preliminary Characterization of a monoclonal antibody that interacts preferentially with the liver enzyme of human alkaline phosphatase"

CHEMICAL ABSTRACTS, vol. 98, no. 7, 14 Feb 1983, Columbus, OH (US); L.MEYER et al., p. 494, no. 51643t : "Production of a monoclonal antibody to human liver alkaline phosphatase"

CHEMICAL ABSTRACTS, vol. 102, no. 13, 01 April 1985, Columbus, OH (US); M.BAILYES et al., p. 287, no. 108548y : "Monoclonal antibodies against human alkaline phosphatases"

CLIN.CHIM.ACTA, no. 126, 1982; pp. 109-117

**Beschreibung**

Die alkalische Phosphatase (Ortho phosphoric monoester phosphohydrolase) katalysiert die Hydrolyse von Phosphorsäure-monoestern bei alkalischen pH-Werten. Dieses Enzym kommt in vielen Geweben vor. Es existieren gewebespezifische Formen (Isoenzyme) beispielsweise in der Leber, in den Knochen, in dem Dünndarm, in den Nieren und in der Placenta. Von diesen Organen werden die Isoenzyme an das Blutplasma abgegeben. Die charakteristischen Eigenschaften der jeweiligen Isoenzyme bleiben beim Übergang vom erzeugenden Organ in das Blutplasma erhalten.

Das Blutplasma gesunder Menschen enthält hauptsächlich das Leber- und Knochen-Isoenzym. Die Bestimmung der Gesamtaktivität der alkalischen Phosphatase sowie insbesondere der Aktivität des Leber- und des Knochen-Isoenzyms ist von besonderer diagnostischer Bedeutung bei der Untersuchung von Krankheiten der Leber und des Knochensystems. Die Erkrankungen bewirken einen Anstieg der alkalischen Phosphataseaktivität im Plasma, da unter diesen Voraussetzungen von der Leber bzw. den Knochen im verstärkten Maße alkalische Phosphatase an das Plasma abgegeben wird. Der Anstieg der alkalischen Phosphataseaktivität des Leber- oder des Knochen-Iosenzyms im Plasma kann demnach dazu dienen, eine Erkrankung dieser Gewebe zu erkennen.

Die Differenzierung des Leber- und des Knochen-Isoenzyms von anderen alkalischen Phosphatase-Isoenzymen, z. B. aus Dünndarm, Placenta, Nieren und Galle, ist ohne weiteres möglich, da sich diese gewebespezifischen Formen in ihren chemischen, physikalischen und immunologischen Eigenschaften deutlich unterscheiden. Verfahren zur Abtrennung und Bestimmung dieser Isoenzyme sind bekannt und beruhen beispielsweise auf dem unterschiedlichen Verhalten der Isoenzyme gegen Inhibitoren, auf Unterschiede in der elektrischen Ladung usw. So können beispielsweise die Unterschiede in der elektrophoretischen Beweglichkeit in alkalischen Puffersystemen zur Trennung dieser Isoenzyme herangezogen werden.

Geringere Unterschiede sind jedoch zwischem dem Leber- und dem Knochen-Isoenzym zu beobachten. Insbesondere unterscheiden sich das Leber- und das Knochen-Isoenzym der alkalischen Phosphatase nur wenig hinsichtlich der Hitzestabilität und in der Antwort auf Inhibitoren (beispielsweise gegenüber Harnstoff). Diese Unterschiede genügen nicht, um in einem Gemisch beider Isoenzyme eine Iosenzym-Form hinreichend und einfach zu quantifizieren. Etwas stärker ausgeprägt sind die Unterschiede in der elektrischen Ladung. Diese werden zur Trennung des Knochen- und des Leber-Isoenzyms und somit zur selektiven Bestimmung der beiden Isoenzyme ausgenutzt. Diese Trennverfahren sowie die darauf beruhenden Bestimmungsmethoden sind arbeits- und zeitaufwendig und erfordern darüberhinaus einen großen apparativen Aufwand.

Antikörper gegen Isoenzyme der alkalischen Phosphatase sind bekannt. Beispielsweise wird in Clin. Chem. Acta 1982, 126, S. 109-117 ein monoklonaler Antikörper gegen das Leberisoenzym der alkalischen Phosphatase beschrieben. Dieser Antikörper weist jedoch eine starke Kreuzreaktivität zum Knochenisoenzym der alkalischen Phosphatase auf. Er ist daher für Verfahren zur Differenzierung zwischen dem Leber- und dem Knochenisoenzym nicht verwendbar.

Es besteht weiterhin Bedarf an einer Möglichkeit, die beiden Isoenzmye auf einfache Weise schnell und zuverlässig soweit trennen bzw. differenzieren zu können, daß mindestens eines der beiden Isoenzyme selektiv bestimmt werden kann.Die Aktivität des jeweils anderen Isoenzyms könnte dann leicht als Differenz aus der nach bekannten Methoden bestimmbaren Gesamt-Aktivität des Gemisches und der gemessenen Knochen- bzw. Leber-Isoenzym-Aktivität ermittelt werden.

Aufgabe der Erfindung war es daher, ein neues Verfahren und Reagenz bereitzustellen, mit dessen Hilfe eine Differenzierung des Leber- und des Knochen-Isoenzyms der alkalischen Phosphatase und eine selektive Bestimmung der differenzierten Isoenzyme in einer Körperflüssigkeit ermöglicht wird. Gelöst wird diese Aufgabe durch das erfindungsgemäße Verfahren, bei dem die Differenzierung mit Hilfe eines monoklonalen Antikörpers gegen das Leber-Isoenzym erfolgt, der nur eine geringe Kreuzreaktion mit dem Knochen-Isoenzym aufweist.

Es konnte gezeigt werden, daß es überraschenderweise gelingt, einen monoklonalen Antikörper zu finden, der überwiegend das Leber-Isoenzym bindet und nur eine geringe Kreuzreaktivität gegenüber dem Knochen-Isoenzym aufweist. Dies war nicht zu erwarten, da gerade das Leber- und das Knochen-Isoenzym in ihrer Eigenschaft so weitgehend ähnlich sind, daß eine immunologische Differenzierung dieser beiden Isoenzyme bisher nicht möglich gewesen ist.

Ein weiterer Gegenstand der Erfindung ist ein neuer monoklonaler Antikörper, der gegen das Leber-Isoenzym gerichtet ist und nur eine geringe Kreuzreaktivität gegenüber dem Knochen-Isoenzym aufweist, erhältlich durch Immunisierung mit dem Knochen-Isoenzym. Die Kreuzreaktivität mit dem Knochen-Isoenzym kann bei 20% oder weniger liegen.

Der erfindungsgemäße monoklonale Antikörper läßt sich in konventioneller Weise erhalten. Hierzu werden Versuchstiere mit hochgereinigter alkalischer Phosphatase aus Knochen immunisiert. B-Lymphozyten der so erhaltenen immunisierten Tiere werden mit transformierenden Agenzien fusioniert. Die so gebildeten Primärkulturen von Hybridzellen werden kloniert. Es werden jeweils die Kulturen weiterverarbeitet, die in einem geeigneten Testverfahren, beispielsweise einem Enzym-Immunoassay (ELISA-Verfahren), positiv gegen das Leber-Isoenzym,

und negativ mit dem Leber-Isoenzym reagieren. Man erhält so eine Hybridoma-Zellinie, die den erfindungsgemäßen monoklonalen Antikörper produziert. Nach bekannten Methoden kann diese Zellinie kultiviert und der von ihr produzierte monoklonale Antikörper isoliert werden.

Die zur Immunisierung besonders geeigneten Tiere sind vor allem Ratten und Mäuse. Die Immunisierung erfolgt mit hochgereinigter alkalischer Phosphatase aus Knochen. Hierzu wird das Enzym, vorzugsweise in Kombination mit einem Adjuvanz in üblicher Weise dem Wirtstier verabreicht. Bevorzugt wird als Adjuvanz Aluminiumhydroxid zusammen mit Bordatella pertussis oder Freundsches Adjuvanz eingesetzt.

Die Immunisierung erfolgt vorzugsweise über mehrere Monate mit mindestens 4 Immunisierungen (Injektion intraperitoneal).

Nach erfolgter Immunisierung werden die B-Lymphozyten der immunisierten Tiere nach üblichen Methoden mit transformierenden Agenzien fusioniert. Beispiel für transformierende Agenzien, die im Rahmen der Erfindung verwendet werden können, sind Myeloma-Zellen, transformierende Viren, wie z. B. Epstein-Barr-Virus oder die in der Deutschen Offenlegungsschrift 32 45 665 beschriebenen Agenzien. Die Fusionierung erfolgt nach dem bekannten Verfahren von Koehler und Milstein (Nature 256 [1975] S. 495 - 997). Die hierbei gebildeten Primärkulturen von Hybridzellen werden, wenn sie Antikörper der gewünschten Spezifität enthalten, in üblicher Weise kloniert, z. B. unter Verwendung eines handelsüblichen Zellsorters oder durch "limiting dilution" und die erhaltenen Klone, die den gewünschten monoklonalen Antikörper bilden, gezüchtet. Aufgrund des krebsartigen Wachsens der Hybridzellen lassen sich diese auf unbestimmte Zeit weiter kultivieren und produzieren den gewünschten monoklonalen Antikörper in beliebiger Menge.

Eine auf solche Weise gewonnene Hybridoma-Zellinie stellt die Zellinie B 4 - 50 dar, die bei der American Type Culture Collection unter der Nummer ATCC 8571 hinterlegt worden ist. Diese Zellinie produziert einen monoklonalen Antikörper, ebenfalls als B 4 - 50 bezeichnet, der gegen das Leber-Isoenzym der alkalischen Phosphatase gerichtet ist und eine Kreuzreaktivität gegenüber dem Knochen-Isoenzym von ca. 20 % aufweist.

Für das erfindungsgemäße Bestimmungsverfahren können die monoklonalen Antikörper als solche oder Fragmente hiervon, die die entsprechenden immunologischen Eigenschaften aufweisen (Fab-Fragmente), verwendet werden. Unter dem Begriff "monoklonaler Antikörper" werden daher sowohl die vollständigen Antikörper als auch die Fragmente verstanden.

Die so erhältlichen monoklonalen Antikörper sind gegen das Leber-Isoenzym der alkalischen Phosphatase gerichtet und zeigen nur eine gewisse Kreuzreaktion gegenüber dem Knochen-Isoenzym. Sie eignen sich daher hervorragend dazu, in einer Probe, die sowohl das Leber- als auch das Knochen-Isoenzym enthält, bevorzugt das Leber-Isoenzym zu binden. Auf diese Weise kann zwischen den beiden Isoenzymen differenziert werden. Sie können selektiv bestimmt werden. Dies erfolgt vorzugsweise, indem die alkalische Phosphatase-Aktivität des gebundenen Isoenzym-Anteils ohne diejenige des gelösten Isoenzym-Anteils ermittelt wird. Anhand einer Eichkurve, die mit bekannten Isoenzym-Konzentrationen erstellt wird, kann der Anteil des Leber-Isoenzyms bestimmt werden. Die Aktivität des jeweils anderen Isoenzyms ergibt sich aus der Differenz der Gesamtaktivität der alkalischen Phosphatase der Probe und der gemessenen Isoenzym-Aktivität.

Die Bestimmung der alkalischen Phosphataseaktivität erfolgt jeweils nach den hierfür bekannten Methoden.

Zur Durchführung des erfindungsgemäßen Verfahrens wird der monoklonale Antikörper vorzugsweise auf einem festen Träger fixiert, beispielsweise auf immunosorptivem Papier, auf aktivierten Glas- oder Latexpartikeln, auf Mikrotiterplatten oder auf der Oberfläche von Kunststoffröhrchen bzw. Schläuchen. Auf diese Weise wird das Isoenzym, gegen das der eingesetzte monoklonale Antikörper gerichtet ist, an den Träger, also die feste Phase, gebunden und kann dort, wie oben beschrieben, bestimmt werden.

Es ist aber auch möglich, das gebundene Enzym von der flüssigen Phase abzutrennen und die restliche alkalische Phosphatase-Aktivität in der flüssigen Phase zu ermitteln. Anhand einer Eichkurve, die wiederum mit bekannten Enzymkonzentrationen erstellt wird, läßt sich auf diese Weise der Knochen-Isoenzym-Anteil bestimmen.

Aus der Differenz der gesamten alkalischen Phosphatase-Aktivität und dem gemessenen Isoenzym-Anteil ergibt sich der Anteil des jeweils anderen Isoenzyms.

Erfindungsgemäß läßt sich auch ein Antikörper-Präparat verwenden, das aus mehreren monoklonalen Antikörpern besteht, die durch mehrere verschiedene Klone produziert worden sind.

Der aus dem monoklonalen Antikörper und seinem Antigen, dem Leber-Isoenzym der alkalischen Phosphatase gebildete Komplex ist löslich. Seine Abtrennung aus der Probeflüssigkeit kann auch derart erfolgen, daß ein zusätzlicher Antikörper gegen den monoklonalen Antikörper (also ein Anti-Antikörper) zugesetzt wird. Hierbei bildet sich ein unlöslicher Komplex aus dem Leber-Isoenzym der alkalischen Phosphatase, dem monoklonalen Antikörper gegen dieses Isoenzym und dem Anti-Antikörper.

Eine zweckmäßige Ausführungsform des erfindungsgemäßen Verfahrens besteht darin, zuerst einen Komplex aus monoklonalem Antikörper und dem Anti-Antikörper zu bilden und diesen dann der zur untersuchenden Probeflüssigkeit zuzusetzen. Alternativ kann man jedoch auch zuerst der Probeflüssigkeit nur den monoklonalen Antikörper und nach einer Inkubationszeit, die zur Bildung des Antigen-Antikörper-Komplexes mit dem Leber-Isoenzym der alkalischen Phosphatase ausreicht, dann den Anti-Antikörper unter Bildung des unlöslichen Komplexes zusetzen. Für die Ausführungsform des erfindungsgemäßen Verfahrens unter Verwendung eines Anti-Antikörpers eignen sich prinzipiell alle gegen den monoklonalen Antikörper gerichteten Anti-Antikörper. Wenn die Erzeugung des erfindungsgemäßen monoklonalen Antikörpers in Mäusen oder Ratten erfolgt, verwendet man bevorzugt vom Schaf gebildete Anti-Antikörper, die gegen den Fc-Teil des monoklonalen Antikörpers gerichtet sind.

Ein weiterer Gegenstand der Erfindung ist ein Reagenz zur Differenzierung des Leber- und des Knochen-Isoenzyms der alkalischen Phosphatase und spezifischen Bestimmung eines der beiden Isoenzyme neben dem jeweils anderen Isoenzym in Körperflüssigkeiten, insbesondere im Serum oder Plasma, das ein System zum Nachweis von alkalischer Phosphatase und ein Mittel zur Differenzierung der beiden Isoenzyme enthält, das dadurch gekennzeichnet ist, daß es als Mittel zur Differenzierung einen monoklonalen Antikörper gegen das Leber-Isoenzym enthält, der mit dem Knochen-Isoenzym nur wenig kreuzreagiert.

Das Reagenz enthält den monoklonalen Antikörper vorzugsweise an einen festen Träger fixiert. Es kann auch den monoklonalen Antikörper in freier Form und zusätzlich einen Anti-Antikörper, der gegen den monoklonalen Antikörper gerichtet ist, enthalten.

Die obigen Ausführungen zum Verfahren und zu dem monoklonalen Antikörper gelten in entsprechender Weise auch für das erfindungsgemäße Reagenz.

Die Erfindung ermöglicht eine einfache und rasche Differenzierung und selektive Bestimmung des Leber- oder des Knochen-Isoenzyms der alkalischen Phosphatase neben dem jeweils anderen Isoenzym in Körperflüssigkeiten. Die Aktivität des jeweils anderen Isoenzyms in der Probeflüssigkeit ergibt sich als Differenz aus der Gesamtaktivität, die nach üblichen Methoden bestimmt werden kann und der erfindungsgemäß ermittelten Aktivität des Leber- oder Knochen-Isoenzyms.

In den beigefügten Abbildungen wird dargestellt:

Abb. 1: Extinktion [mE] in einem ELISA mit dem monoklonalen Antikörper B 4 - 50 als Funktion der eingesetzten Konzentration in [U/1] an Leber(◊)- und Knochen(▲)-Isoenzym der alkalischen Phosphatase.

Abb. 2: Eichkurve zur Bestimmung des Gehaltes [%] von Leber-Isoenzym der alkalischen Phosphatase in Humanseren. P1 und P2 sind Serumproben (siehe Beispiel 5).

Die folgenden Beispiele erläutern die Erfindung.

Beispiel 1:

A) Immunisierung von Balb/c-Mäusen mit alkalischer Phosphatase aus menschlichen Knochen

Balb/c-Mäuse werden mit 100 μg alkalischer Phosphatase aus menschlichen Knochen in komplettem Freundschen Adjuvanz intraperitoneal immunisiert. In ca. achtwöchigem Rhythmus wird mit je 50 μg Enzym in inkomplettem Freundschen Adjuvanz weiterimmunisiert. Vier Tage vor Fusion wird die letzte Immunisierung mit 50 μg Enzym in physiologischer Kochsalzlösung intravenös durchgeführt.

B) Fusion der Maus-Milzzellen mit Myelomazellen

Die Fusion der Milzzellen nach A) immunisierter Balb/c-Mäuse mit Ag8.653 (ATCC CRL 1580) oder SPL/D (ATCC CRL 1581) Myelomzellen wird nach dem Standardverfahren gemäß W. B. Foster et al. Thrombosis Research 28 (1982) S. 649 - 661 durchgeführt. Das Fusionsverhältnis Milzzu Myselomzellen ist 5 : 1. Die Fusionsprodukte werden auf 24er-Kulturschalen ausgesäht und mit $5.10^4$ Peritonealexsudatzellenpro Kulturnapf gefüttert. Positive Primärkulturen werden 3 bis 4 Wochen nach Fusion durch "limiting dilution" kloniert. Die Zellen werden in 96er-Kulturplatten abgelegt und mit $2.10^4$ Peritonealexsudatzellen gefüttert. Die resultierenden Klone werden auf ihre Eigenschaft hin, Antikörper zu produzieren, untersucht und positive Klone werden entweder in flüssigem Stickstoff gelagert oder zur MAK-Produktion in Maus Ascites herangezogen. Auf diese Weise läßt sich die Hybridoma-Zellinie B4 - 50 gewinnen, die bei der American Type Culture Collection unter der Nummer ATCC 8571 hinterlegt worden ist.

C) Produktion des monoklonalen Antikörpers über Maus Ascites

Weibliche Balb/c-Mäuse werden zweimal mit 0,5 ml Pristan intraperitoneal im 7tägigen Abstand gespritzt. Pro Maus werden ca. $5.10^6$ Hybridomazellen, die gemäß B) gewonnen worden sind, intraperitoneal inokkuliert. Nach 8 bis 14 Tagen wird 1 bis 3 Mal Ascites gezapft. Dieser wird über eine Anionenaustauscher-Säule gereinigt und man erhält den reinen monoklonalen Antikörper. Mit Hilfe der Hybridoma-Zellinie B 4 - 50 wird der monoklonale Antikörper B 4 - 50 gewonnen, der gegen das Leber-Isoenzym gerichtet ist und eine Kreuzreaktivität gegenüber dem Knochen-Isoenzym von 20 % aufweist. Er gehört der Subklasse IgG 1 K an.

Beispiel 2: Enzymimmunoassay (ELISA) auf alkalische Phosphatase bindende Antikörper

Um die Anwesenheit und Spezifität alkalischer Phosphatase bindender Antikörper im Serum immunisierter Mäuse oder in Kulturüberstand der Hybridzellen oder in Ascites zu erkennen, wird ein ELISA als Testprinzip verwendet:

100 µl polyklonaler Kaninchen-anti-Mausantikörper Antikörper wird in einer Konzentration von 10 µg/ml für 18 h bei 4° C in 0,05 M Natriumcarbonatpuffer, pH = 9,3, in jede Vertiefung einer "Dynatech Microfluor" Mikrotiterplatte gegeben. Anschließend wird mit 1 % Rinderserumalbumin in 50 mM Trispuffer, pH = 7,1, nachbeschichtet. 100 µl Probe pro Vertiefung (Serum, Kulturüberstand oder Ascites, jeweils in geeigneter Verdünnung) wird für 5 h bei Raumtemperatur inkubiert. Anschließend wird mit 100 µl humaner Knochen alkalischer Phosphatase oder mit humaner Leber alkalischer Phosphatase in einer Konzentration von 100 U/l (bestimmt mit handelsüblichem Reagenz mit Paranitrophenylphosphat als Substrat, 37° C) in Trispuffer, 50 mM, pH = 7,1, für 4 h bei Raumtemperatur inkubiert. Nach ausgiebigem Waschen mit Trispuffer, 50 mM, pH = 7,1, 0,9 % NaCl, wird das Reagenz 4-Methylumbelliferylphosphat einer Konzentration von 3,8 mM in 1,03 M Diethanolaminpuffer, pH = 10,4 mit 0,5 mM $MgCl_2$, zugegeben. Die Fluoreszenz des freigesetzten 4-Methylumbelliferons wird in einem "Dynatech Microfluorreader" nach 30 - 60 Minuten gemessen. Die Kreuzreaktivität des monoklonalen Antikörpers zwischen Knochen und Leber alkalischer Phosphatase errechnet sich aus dem Verhältnis der gemessenen Fluoreszenzintensitäten.

Beispiel 3: Bestimmung des Gesamtgehaltes an alkalischer Phosphatase in Flüssigkeiten, speziell in menschlichem Serum

3 ml eines handelsüblichen Testreagenzes auf alkalische Phosphatase (beispielsweise von Boehringer Mannheim, Kat. Best. Nr. 415 278) werden bei 37° C in einem Photometer temperiert. Nach Zugabe von 50 µl Probe (Serum oder Lösungen alkalischer Phosphatasen) wird die Extinktionszunahme bei 405 nm als E/Minute bestimmt. Nach Multiplikation der Reaktionsgeschwindigkeit mit dem Faktor 3300 erhält man die Gesamtalkalische-Phosphatase-Konzentration der Probe in der Einheit U/l.

Beispiel 4: Bestimmung der Affinität des Leber- und des Knochen-Isoenzyms der alkalischen Phosphatase zum monoklonalen Antikörper B 4 - 50

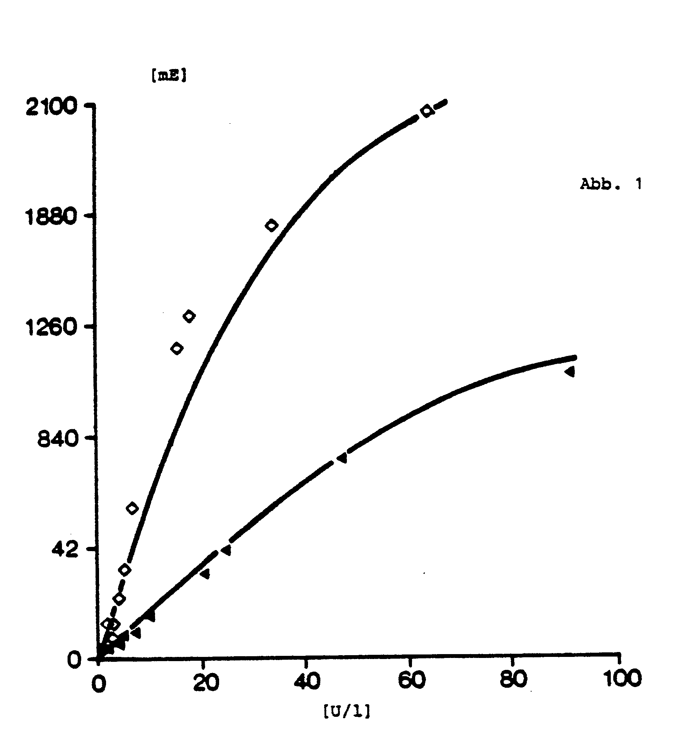

ELISA-Platten der Firma Nunc werden mit 10 µg/ml monoklonalem Antikörper B 4 - 50 (100 µl pro Vertiefung) in 0,05 M Natriumcarbonatpuffer, pH = 9,5 bei 4° C 18 h beschichtet. Nach Nachbeschichtung (0,01 M Natriumphosphatpuffer, pH = 7,4, 0,14 M Natriumchlorid, 1 % Rinderserumalbumin, 2 h, Raumtemperatur) wird für 2 h bei 30° C mit humaner Leber alkalischer Phosphatase bzw. mit humaner Knochen alkalischer Phosphatase in einer Konzentration von 5 - 100 U/l (bestimmt nach Beispiel 5) in 0,05 M Trispuffer, pH = 7,1, 0,9 % Natriumchlorid inkubiert. Anschließend wird ausgiebig gewaschen (0,05 M Trispuffer, pH = 7,1, 0,14 M Natriumchlorid, 0,05 % Tween-20) und es werden die Platten je Vertiefung mit 100 µl einer geeigneten Substratlösung für die alkalische Phosphatase (beispielsweise das handelsübliche Substrat Paranitrophenylphosphat, Boehringer Mannheim Kat. Best. 415 278) für 30 bis 60 Minuten inkubiert (Raumtemperatur). Die Extinktionen wurden danach mit einem "Dynatech Mikroelisareader" ausgewertet. Abbildung 1 zeigt die gemessenen Extinktionen als Funktion der eingesetzten alkalischen Phosphat-Mengen. Es zeigt sich, daß der monoklonale Antikörper B 4 - 50 die Knochen-alkalische-Phosphatase schlechter bindet als die Leber-alkalische-Phosphatase.

Beispiel 5: Bestimmung der menschlichen Leber- und Knochen-Isoenzyme der alkalischen Phosphatase in Serumproben

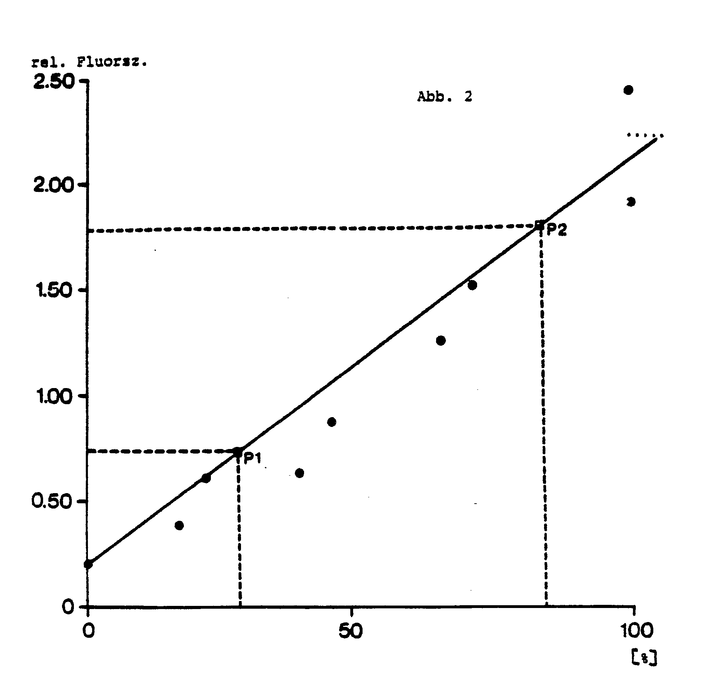

"Dynatech Microfluor" Mikrotiterplatten werden halb mit erfindungsgemäßem monoklonalem Antikörper (B 4 - 50) gegen das humane Leber-Isoenzym der alkalischen Phosphatase und halb mit einem gereinigten üblichen unspezifischen monoklonalen Antikörper (hier mit B 4 - 78 bezeichnet) gegen humane alkalische Phosphatase beschichtet. (100 µl/Vertiefung), 10 µg/ml, 0,05 M Natriumcarbonatpuffer, pH = 9,5, 4° C, 18 h). Anschließend wird die Platte ausgiebig mit Puffer gewaschen (0,01 M Natriumphosphatpuffer, pH = 7,4, 0,14 M NaCl, 0,05 % Tween-20, 0,02 % $NaN_3$). Reines humanes Leber-Isoenzym der alkalischen Phosphatase (100 % L-AP), reine humane alkalische Phosphatase aus Knochen (0 % L-AP) bzw. definierte Mischungen der beiden Isoenzyme im Konzentrationsbereich von 50 - 100 U/l (bestimmt nach Beispiel 3) werden einmal in Vertiefungen mit dem unspezifischen Antikörper und einmal in Vertiefungen mit dem spezifischen Antikörper gegeben. Die Serumproben P1 und P2 mit unbekanntem alkalischen Phosphatase-Gehalt werden verdünnt (mit 0,05 M Trispuffer. pH = 7,1, 0,9 % NaCl) auf eine Konzentration von 50 - 100 U/l (bestimmt nach Beispiel 5) und ebenfalls einmal auf Vertiefungen mit spezifischem und einmal auf Vertiefungen mit unspezifischem Antikörper verteilt. Es wird 2 h bei 30° C inkubiert. Anschließend wird ausgiebig gewaschen (0,05 M Trispuffer, pH = 7,4, 0,14 M NaCl, 0,05 % Tween-20). Danach werden je Vertiefung 100 $\mu$l einer 3,8 mM 4-Methylumbelliferylphosphatlösung in 1,03 M Diethanolaminpuffer mit 0,5 mM $MgCl_2$, pH = 10,4, zugegeben. Nach 30 - 60 Minuten wird die Fluoreszenz des 4-Methylumbelliferons im "Dynatech Microfluorreader" gemessen. Es ergibt sich die in Abbildung 2 wiedergegebene Eichkurve, wenn die Fluoreszenz der in den Vertiefungen mit L-AP spezifischem monoklonalem Antikörper relativiert zu den Fluoreszenzintensitäten mit unspezifisch monoklonalem Antikörper aufgetragen werden gegen den %-Gehalt an humaner Leber alkalischer Phosphatase der Standardlösungen. Aus den relativierten Fluoreszenzintensitäten der Proben läßt sich aus der Eichkurve der %-Gehalt an Leber-Isoenzym der alkalischen Phosphatase in der Probe ablesen (P1 und P2 in Abbildung 2). Aus diesem Prozentgehalt und der Gesamt-alkalische-Phosphatase-Konzentration (bestimmt nach Beispiel 5) läßt sich die Konzentration der L-AP und K-AP in den Proben in der Einheit U/l berechnen.

Die Probe P1 enthält demnach 29 % L-AP und 71 % K-AP; die Probe P2 85 % L-AP und 15 % K-AP.

**Patentansprüche**

1. Verfahren zur Differenzierung des Leber- und Knochen-Isoenzyms der alkalischen Phosphatase und selektiven Bestimmung der differenzierten Isoenzyme durch Messen der alkalischen Phosphatase-Aktivität, dadurch gekennzeichnet, daß die Differenzierung mit Hilfe eines monoklonalen Antikörpers gegen das Leber-Isoenzym der alkalischen Phosphatase mit geringer Kreuzreaktivität gegenüber dem Knochen-Isoenzym erfolgt.

2. Verfahren gemäß Anspruch 1, dadurch gekennzeichnet, daß die Kreuzreaktivität des monoklonalen Antikörpers gegen das Leber-Isoenzym gegenüber dem Knochen-Isoenzym 20 % oder weniger beträgt.

3. Reagenz zur Differenzierung des Leber- und des Knochen-Isoenzyms der alkalischen Phosphatase und spezifischer Bestimmung eines der beiden Isoenzyme neben dem jeweils anderen Isoenzym, enthaltend ein System zum Nachweis von alkalischer Phosphatase und ein Mittel zur Differenzierung der beiden Isoenzyme, dadurch gekennzeichnet, daß es als Mittel zur Differenzierung der beiden Isoenzyme einen monoklonalen Antikörper gegen das Leber-Isoenzym enthält, der mit dem Knochen-Isoenzym nur wenig kreuzreagiert.

4. Reagenz gemäß Anspruch 3, dadurch gekennzeichnet, daß die Kreuzreaktivität des monoklonalen Antikörpers gegen das Leber-Isoenzym gegenüber dem Knochen-Isoenzym 20 % oder weniger beträgt.

5. Monoklonaler Antikörper gegen ein Isoenzym der alkalischen Phosphatase, dadurch gekennzeichnet, daß er gegen das Leber-Isoenzym gerichtet ist und mit dem Knochen-Isoenzym nur wenig kreuzreagiert , erhältlich durch Immunisierung mit dem Knochen-Isoenzym.

6. Verfahren zur Herstellung eines monoklonalen Antikörpers gegen das Leber-Isoenzym der alkalischen Phosphatase, dadurch gekennzeichnet, daß zur Immunisierung das Knochen-Isoenzym der alkalischen Phosphatase verwendet wird, nach Zellen gesucht wird, die einen monoklonalen Antikörper bilden, der gegen das Leber-Isoenzym gerichtet ist und nur geringe Kreuzreaktivität mit dem Knochen-Isoenzym aufweist, und den Antikörper aus diesen Zellen gewinnt.

**Claims**

1. Process for the differentiation of the liver and bone isoenzyme of alkaline phosphatase and selective determination of the differentiated isoenzymes by measurement of the alkaline phosphatase activity, characterised in that the differentiation takes place with the help of a monoclonal antibody against the liver isoenzyme of alkaline phosphatase with low cross-reactivity towards the bone isoenzyme.

2. Process according to claim 1, characterised in that the cross-reactivity of the monoclonal anti-

3. Reagent for the differentiation of the liver and of the bone isoenzyme of alkaline phosphatase and specific determination of one of the two isoenzymes in the presence of the other isoenzyme, containing a system for the detection of alkaline phosphatase and an agent for the differentiation of the two enzymes, characterised in that, as agent for the differentiation of the two isoenzymes, it contains a monoclonal antibody against the liver isoenzyme which cross-reacts only a little with the bone isoenzyme.

4. Reagent according to claim 3, characterised in that the cross-reactivity of the monoclonal antibody against the liver isoenzyme amounts to 20% or less with regard to the bone isoenzyme.

5. Monoclonal antibody against an isoenzyme of alkaline phosphatase, characterised in that it is directed against the liver isoenzyme and cross-reacts only a little with the bone isoenzyme, obtainable by immunisation with the bone isoenzyme.

6. Process for the production of a monoclonal antibody against the liver isoenzyme of alkaline phosphatase, characterised in that, for the immunisation, the bone isoenzyme of alkaline phosphatase is used, a search is made for cells which form a monoclonal antibody which is directed against the liver isoenzyme and only display low cross-reactivity with the bone isoenzyme and the antibody is recovered from these cells.

**Revendications**

1. Procédé pour différencier les isoenzymes de la phosphatase alcaline du foie et des os et pour déterminer sélectivement les isoenzymes différenciées par mesure de l'activité de phosphatase alcaline, caractérisé en ce que la différenciation est effectuée à l'aide d'un anticorps monoclonal dirigé contre l'isoenzyme de la phosphatase alcaline du foie, et possédant une faible transréactivité vis-à-vis de l'isoenzyme des os.

2. Procédé selon la revendication 1, caractérisé en ce que la transréactivité de l'anticorps monoclonal dirigé contre l'isoenzyme du foie est de 20 % ou moins vis-à-vis de l'isoenzyme des os.

3. Réactif pour différencier les isoenzymes de la phosphatase alcaline du foie et des os et pour déterminer spécifiquement une des deux isoenzymes en plus de l'autre isoenzyme, contenant un système pour la détection de la phosphatase alcaline et un agent pour différencier les deux isoenzymes, caractérisé en ce que, comme agent pour différencier les deux isoenzymes, il contient un anticorps monoclonal dirigé contre l'isoenzyme du foie, qui ne présente qu'une faible transréactivité vis-à-vis de l'isoenzyme des os.

4. Réactif selon la revendication 3, caractérisé en ce que la transréactivité de l'anticorps monoclonal dirigé contre l'isoenzyme du foie est de 20 % ou moins vis-à-vis de l'isoenzyme des os.

5. Anticorps monoclonal dirigé contre une isoenzyme de la phosphatase alcaline, caractérisé en ce qu'il est dirigé contre l'isoenzyme du foie et ne présente qu'une faible transréactivité vis-à-vis de l'isoenzyme des os, et peut être obtenu par immunisation à l'aide de l'isoenzyme des os.

6. Procédé pour la préparation d'un anticorps monoclonal dirigé contre l'isoenzyme de la phosphatase alcaline du foie, caractérisé en ce que pour l'immunisation, on utilise l'isoenzyme de la phosphatase alcaline des os, on recherche les cellules formant un anticorps monoclonal dirigé contre l'isoenzyme du foie et ne présentant qu'une faible transréactivité vis-à-vis de l'isoenzyme des os, et l'on obtient l'anticorps à partir de ces cellules.

Abb. 1

Abb. 2